# EUROPÄISCHE PATENTANMELDUNG

(11) **EP 1 410 817 A1**
(43) Veröffentlichungstag der Anmeldung: **21.04.2004**
(21) Anmeldenummer: 03021968.7
(22) Anmeldetag: 30.09.2003
(51) Int. Cl.: A61M 5/34

(54) **Spritzenzylinder**

(30) Priorität: 15.10.2002 DE 20215807 U
(71) Anmelder: TRANSCOJECT GESELLSCHAFT FÜR MEDIZINISCHE GERÄTE mbH & CO. KG, 24539 Neumünster (DE)
(72) Erfinder: Heinz, Jochen, Dr., 24146 Kiel (DE); Schilling, Dieter, 24613 Aukrug-Innien (DE)
(74) Vertreter: Vollmann, Heiko, Dipl.-Ing.

(57) **Zusammenfassung**

Der Spritzenzylinder (1) ist zu einer Seite offen für die Aufnahme eines Kolbens ausgebildet und weist zur anderen Seite einen Spritzenanschluss in Form eines Luerlockanschlusses (2, 3, 4) auf. Der Luerlockanschluss wird durch einen Lueranschluss (2) und einen diesen umgebenden Zylinderabschnitt (3) mit Innengewinde (4) gebildet. Am Außenumfang des Zylinderabschnitts (3) ist ein zum anschlussseitigen Ende (5) hin verjüngend zulaufender Ring (6) angeordnet (Fig. 1).

## Beschreibung

Die Erfindung betrifft einen Spritzenzylinder, insbesondere für eine vorfüllbare oder vorgefüllte Spritze gemäß den im Oberbegriff des Anspruchs 1 angegebenen Merkmalen.

Spritzenzylinder dieser Art sind aus Glas oder inzwischen auch aus Kunststoff gefertigt und weisen typischerweise einen Luerlockanschluss auf, welcher den Verschlussstopfen hält, mit dem der vorgefüllte Spritzenzylinder verschlossen wird und an dem nach Entfernen des Verschlussstopfens eine Kanüle, ein Schlauch oder dergleichen angeschlossen werden kann. Ein solcher Spritzenzylinder ist beispielsweise aus EP 0 716 860 A2 bekannt.

Nachteilig bei diesen mit Luerlockanschluss versehenen Spritzenzylindern ist, dass sie einfach und unkompliziert nur mit Luer bzw. Luerlock kompatiblen Anschlusssystemen verbunden werden können. Häufig stellt sich jedoch das Problem, dass beispielsweise ein Schlauch anzuschließen ist, an dem ein solcher Anschluss nicht vorhanden ist. Die Montage und Abdichtung gestaltet sich dann recht aufwändig.

Vor diesem Hintergrund liegt der Erfindung die Aufgabe zugrunde, einen Spritzenzylinder mit Luerlockanschluss zu schaffen, der kostengünstig herstellbar und möglichst vielseitig anwendbar ist.

Diese Aufgabe wird gemäß der Erfindung durch die in Anspruch 1 angegebenen Merkmale gelöst. Vorteilhafte Ausgestaltungen der Erfindungen sind in Unteransprüchen der nachfolgenden Beschreibung sowie den Fig. angegeben.

Grundgedanke der vorliegenden Erfindung ist es, an der Außenseite des Luerlockanschlusses Mittel vorzusehen, die es erlauben, einen Schlauch direkt anzuschließen und die insbesondere das Aufschieben und Abdichten erleichtern. Hierzu sieht die Erfindung mindestens einen oder auch mehrere hintereinander angeordnete und zum anschlussseitigen Ende des Spritzenzylinders hin verjüngend zulaufende Ringe vor, welche einerseits das Aufschieben eines Schlauchendes erleichtern und andererseits aufgrund der erhöhten Flächenpressung zwischen dem Schlauch und dem erhabenen Teil des Ringes eine gute Abdichtung sicherzustellen. Bevorzugt sind dabei Spritzenzylinder, Luerlockanschluss und Ring als Kunststoffspritzgussteile gefertigt, sei es einstückig oder mehrstückig. Dieser verjüngend zulaufende Ring kann darüber hinaus auch Teil einer Rastverbindung bilden, auf die ein Anschlussstück aufgeschoben wird oder mittels der eine Schutzkappe befestigt wird.

Die einstückige Ausbildung wird insbesondere bei hohen Stückzahlen hinsichtlich der Fertigungskosten zu bevorzugen sein, wobei der Werkzeugaufbau komplizierter und damit aufwändiger ist.

Bevorzugt ist der Ring konisch ausgebildet und weist eine umlaufende Kante auf, welche die eigentliche Dichtkante zwischen Schlauch und Ring bildet. Diese Kante muss keine Kante im geometrischen Sinne sein, sondern kann gerundet oder auch durch einen vergleichsweise schmalen erhabenen Ringabschnitt gebildet sein.

Um das Aufschieben eines Schlauches auf den Zylinderabschnitt des Luerlockanschlusses zu erleichtern, ist es von Vorteil, wenn der Ring mit seinem verjüngend zulaufenden Ende stufenlos in den Zylinderabschnitt übergeht, so dass beim Aufschieben des Schlauches dieser durch den Ring quasi selbsttätig aufgeweitet und geführt wird.

Bevorzugt ist der Ring ganz am anschlussseitigen Ende des Luerlockanschlusses angeordnet, so dass er bündig mit der Stirnseite des Zylinderabschnitts abschließt. Hinter diesem mit dem Zylinderabschnitt bündig abschließenden Ring können gegebenenfalls noch weitere Ringe angeformt sein.

Der Werkzeugaufbau kann durch eine Ausbildung wesentlich vereinfacht werden, bei der der Spritzenzylinder zusammen mit dem Lueranschluss einstückig als Kunststoffspritzgussteil ausgebildet sind und der Zylinderabschnitt des Luerlockanschlusses mit dem Ring einstückig als Kunststoffspritzgussteil ausgebildet sind. Die beiden Bauteile werden dann nach dem Spritzen zusammengefügt, und zwar stoffschlüssig, bevorzugt durch Schweißen, miteinander verbunden. Werkzeugtechnisch ist eine solche Ausbildung deshalb von Vorteil, da die Einzelwerkzeuge ohne Hinterschnitt auskommen, also vergleichsweise einfach aufgebaut werden können. Diese Ausführung ist insbesondere in Verbindung mit einem Originalitätsverschluss von Vorteil, da dann nämlich der am eigentlichen Spritzenzylinder mittels Ultraschall oder anderweitig anzuschweißende Zylinderabschnitt gleichzeitig Teil des Originalitätsverschlusses bilden kann. Alternativ kann auch nur der Ring Teil des Originalitätsverschlusses bilden, der dann in geeigneter Weise mit dem Zylinderabschnitt zu verbinden ist. Auch dies erfolgt bevorzugt durch Schweißen.

Der erfindungsgemäße Spritzenzylinder besteht vorzugsweise aus Polyolefinen, bevorzugt Polypropylen (PP), Cykloolefinpolymeren (COP) oder anderen Barrierekunststoffen. Diese Werkstoffe eignen sich in besonderer Weise zur Herstellung der vorgenannten Spritzgussteile, sie sind zudem gut verschweißbar.

Die Erfindung ist nachfolgend anhand von in der Zeichnung dargestellten Ausführungsbeispielen näher erläutert. Es zeigen:
- Fig. 1: das anschlussseitige Ende eines Spritzenzylinders mit Luerlockanschluss und Ring gemäß der Erfindung im Längsschnitt,
- Fig. 2: eine Ausführungsvariante, bei der der Zylinderabschnitt und Ring als gesonderte Bauteile ausgebildet sind in Darstellung nach Fig. 1,
- Fig.3: eine dritte Ausführungsvariante, bei der Ring und Verschlusskappe einstückig ausgeführt sind in Darstellung nach Fig. 1 und
- Fig. 4: in vergrößerter Darstellung eine Einzelheit aus Fig. 3.

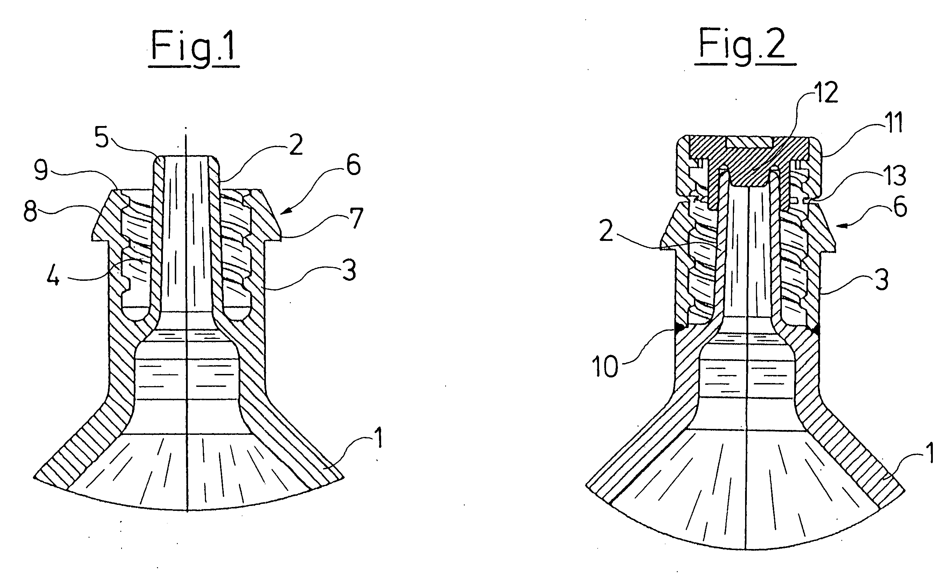

Das in Fig. 1 dargestellte anschlussseitige Ende eines Spritzenzylinders 1, dessen anderes Ende durch einen Kolben in an sich bekannter Weise abschließbar ist, geht bündig in einen Spritzenanschluss über, welcher aus einem Lueranschluss besteht, der von einem Zylinderabschnitt 3 mit einem Innengewinde 4 umgeben ist, so dass sich ein Luerlockanschluss ergibt. An der Außenseite des Zylinderabschnitts 3 ist ein zum anschlussseitigen Ende 5 verjüngend zulaufender Ring 6 angeordnet, der in der Ausführung nach Fig. 1 einstückig mit dem Spritzenzylinder 1, dem Lueranschluss 2 und dem Zylinderabschnitt 3 ausgebildet ist.

Der Ring 6 weist einen schmalen im Wesentlichen zylindrischen Abschnitt 7 auf, der auch gerundet oder als Kante ausgebildet sein kann und Dichtfunktion (zu einem weiter unten beschriebenen Schlauch) übernimmt. Von diesem zylindrischen Abschnitt 7 erstreckt sich ein zum Ende 5 hin konisch zulaufender Abschnitt 8, der mit der Stirnseite 9 des Zylinderabschnitts 3 endet. Dabei ist die Formgebung so, dass der Ring 6 mit seinem konisch zulaufenden Abschnitt 8 bündig in den Außenumfang des Zylinderabschnitts 3 übergeht, d. h. auf etwa einen Umfang zurückgeht, die dem des Zylinderabschnitts 3 entspricht. Je nach Wandstärke kann der Umfang des konisch zulaufenden Abschnitts 8 am anschlussseitigen Ende 5 auch geringer sein als der des Zylinderabschnitts 3 im übrigen Bereich.

Der Ring 6 dient im Wesentlichen dazu, einen elastischen Schlauch unmittelbar am Ende des Spritzenzylinders 1 zu befestigen, dabei dient die Querschnittsverjüngung des Ringes 6 zum Ende 5 hin im Wesentlichen zum Erleichtern des Aufschiebens eines Schlauchs, wohingegen der Abschnitt 7 zur Erzielung einer guten Dichtwirkung zwischen Schlauch und Anschluss vorgesehen ist. In diesem Bereich wird die größte Flächenpressung zwischen dem elastisch aufgeweiteten Schlauch und dem insofern als starr anzusehenden Ring 6 und damit Dichtwirkung erzielt.

Zwar kann ein Spritzenzylinder, wie er vorstehend anhand von Fig. 1 beschrieben worden ist, einstückig hergestellt werden, doch weist der Werkzeugaufbau aufgrund des vorhandenen Hinterschnitts sowohl an der Innen- als auch an der Außenseite des Zylinderabschnitts 3 eine gewisse Komplexität auf, die vermieden werden kann, wenn, wie anhand von Fig. 2 dargestellt, Spritzenzylinder 1 und Lueranschluss 2 als Spritzgussteil einerseits und Zylinderabschnitt 3 mit Ring 6 als weiteres, gesondertes Spritzgussteil aus Kunststoff gefertigt werden. Die Bauteile sind dann durch Ultraschall-Schweißen stoffschlüssig miteinander verbunden worden, die umlaufende Schweißnaht ist in Fig. 2 mit 10 beziffert.

Bei der anhand von Fig. 2 dargestellten Ausführungsvariante ist an den Zylinderabschnitt 3 mit Ring 6 weiterhin eine Verschlusskappe 11 einstückig angeformt. In diese Verschlusskappe 11 ist formschlüssig eingegliedert oder gegebenenfalls auch eingespritzt ein Verschlussstopfen 12, welcher das freie Ende des Lueranschlusses sowohl an der Innen- als auch an der Außenseite umgreift und somit dicht abschließt. Zwischen der Verschlusskappe 11 und dem Zylinderabschnitt 3 sind dünne Stege 13 gebildet, die eine Sollbruchstelle für einen späteren Originalitätsverschluss bilden.

Das durch den Zylinderabschnitt 3 mit Ring und Verschlusskappe 11 gebildete Spritzgussteil wird nach Einfügen des Verschlussstopfens 12 auf den Lueranschluss 2 bzw. das Ende des Spritzenzylinders 1 in der in Fig. 2 dargestellten Stellung aufgesetzt und umlaufend verschweißt, so dass die Schweißnaht 10 gebildet ist. Der Spritzenzylinder 1 wird dann befüllt und durch einen Kolben dicht verschlossen. Zum Gebrauch wird die Verschlusskappe 11 abgezogen, wodurch die Stege 13 zerstört und die Kappe 11 zusammen mit dem darin befindlichen Verschlussstopfen 12 entfernt wird. Der so geöffnete Spritzenzylinder 1 steht dann zum Anschluss am Lueranschluss 2, gegebenenfalls in Form eines Luerlockanschlusses unter Ausnutzung des Innengewindes 4 sowie alternativ auch als Schlauchanschluss über den Außenumfang des Zylinderabschnitts 3 mit darauf befindlichem Ring 6 zur Verfügung.

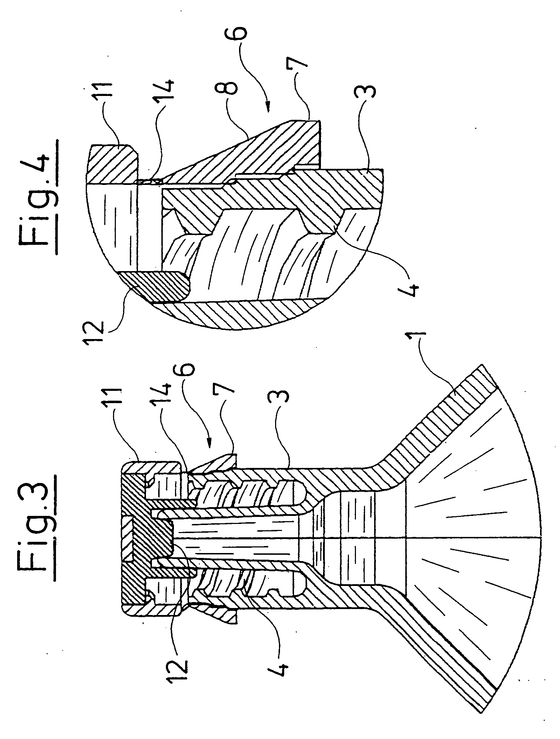

Die Ausführung gemäß Fig. 3 unterscheidet sich von der vorbeschriebenen im Wesentlichen dadurch, dass Spritzenzylinder 1, Lueranschluss 2 und Zylinderabschnitt 3 als einstückiges Spritzgussbauteil einerseits und Ring 6 sowie Verschlusskappe 11 gegebenenfalls mit Verschlussstopfen 12 eingeformt als Spritzgussbauteil andererseits gebildet sind. Zwischen Verschlusskappe 11 und Ring 6 ist eine dünne Wandung 14 vorgesehen, die wie die Stege 13 in Fig. 2 eine Sollbruchstelle für einen Originalitätsverschluss bilden.

Im Bereich des Ringes 6 sind die Außenseiten des Zylinderabschnitts 3 sowie die Innenseite des Ringes 6 jeweils im Wesentlichen komplementär zueinander abgestuft ausgebildet (siehe Fig. 4), wobei der Ring 6 auf das freie Ende des Zylinderabschnitts 3 aufgepresst ist, so dass dieser kraftschlüssig gehalten wird. Alternativ kann auch hier eine Schweißung vorgesehen sein, um diese Bauteile stoffschlüssig miteinander zu verbinden. Die Funktion entspricht im Wesentlichen der anhand von Fig. 2 Vorbeschriebenen.

### Bezugszeichen

- 1: Spritzenzylinder
- 2: Lueranschluss
- 3: Zylinderabschnitt
- 4: Innengewinde
- 5: anschlussseitiges Ende
- 6: Ring
- 7: zylindrischer Abschnitt
- 8: konisch zulaufender Abschnitt
- 9: Stirnseite
- 10: Schweißnaht
- 11: Verschlusskappe
- 12: Verschlussstopfen
- 13: Stege
- 14: Wandung

## Patentansprüche

1. Spritzenzylinder, insbesondere für eine vorfüllbare oder vorgefüllte Spritze, der zu einer Seite offen für die Aufnahme eines Kolbens ist und der zur anderen Seite in einen Spritzenanschluss in Form eines Luerlockanschlusses (2, 3, 4) mündet, wobei der Luerlockanschluss durch einen Lueranschluss (2) und einen diesen umgebenden Zylinderabschnitt (3) mit Innengewinde (4) gebildet ist, **dadurch gekennzeichnet, dass** am Außenumfang des Zylinderabschnitts (3) mindestens ein zum anschlussseitigen Ende (5) hin verjüngend zulaufender Ring (6) angeordnet ist.

2. Spritzenzylinder nach Anspruch 1, **dadurch gekennzeichnet, dass** der Ring (6) einstückig mit dem Zylinderabschnitt (3) ausgebildet ist.

3. Spritzenzylinder nach einem der vorhergehenden Ansprüche, **dadurch gekennzeichnet, dass** der Ring (6) konisch ausgebildet ist und eine umlaufende Kante (7) aufweist.

4. Spritzenzylinder nach einem der vorhergehenden Ansprüche, **dadurch gekennzeichnet, dass** der Ring (6) mit seinem verjüngend zulaufenden Ende stufenlos in den Zylinderabschnitt (3) übergeht.

5. Spritzenzylinder nach einem der vorhergehenden Ansprüche, **dadurch gekennzeichnet, dass** der Ring (6) bündig mit der Stirnseite (9) des Zylinderabschnitts (3) abschließt.

6. Spritzenzylinder nach einem der vorhergehenden Ansprüche, **dadurch gekennzeichnet, dass** der zur Aufnahme des Spritzenkolbens vorgesehene Zylinder (1) mit dem Lueranschluss (2) sowie der Zylinderabschnitt (3) mit dem Ring (6) jeweils einstückig als Kunststoffspritzgussteil ausgebildet sind, und dass die beiden Bauteile stoffschlüssig, vorzugsweise durch Schweißen, miteinander verbunden sind.

7. Spritzenzylinder nach einem der vorhergehenden Ansprüche, **dadurch gekennzeichnet, dass** der zur Aufnahme des Spritzenkolbens vorgesehene Zylinder (1) mit dem Lueranschluss (2) und dem Zylinderabschnitt (3) einstückig als Kunststoffspritzgussteil ausgebildet sind und dass der Ring (6) stoff- oder kraftschlüssig, vorzugsweise durch Schweißen mit dem Zylinderabschnitt (3) verbunden ist.

8. Spritzenzylinder nach einem der vorhergehenden Ansprüche, **dadurch gekennzeichnet, dass** der Ring (6) Teil eines Originalitätsverschlusses (13; 14) bildet.

9. Spritzenzylinder nach einem der vorhergehenden Ansprüche, **dadurch gekennzeichnet, dass** er aus Polyolefinen besteht, bevorzugt aus Polypropylen (PP), oder Cykloolefinpolymeren (COP).
